# EUROPÄISCHE PATENTSCHRIFT

(11) **EP 0 595 144 B1**
(45) Veröffentlichungstag und Bekanntmachung des Hinweises auf die Patenterteilung: **20.05.1998**
(21) Anmeldenummer: 93116774.6
(22) Anmeldetag: 18.10.1993
(51) Int. Cl.: C07C 69/533, C09K 19/30, C09K 19/34, C07D 239/30, C07D 213/30, G02F 1/13

(54) **Cyclohexyl-alkenoate als Komponente flüssigkristalliner Gemische**
Cyclohexyl alkenoates as components of liquid crystalline mixtures
Alcènoates de cyclohexyles comme constituants de mélanges de cristaux liquides

(30) Priorität: 30.10.1992 CH 3391/92
(43) Veröffentlichungstag der Anmeldung: 04.05.1994
(73) Patentinhaber: MERCK PATENT GmbH, 64293 Darmstadt (DE)
(72) Erfinder: Kelly, Stephen, CH-4313 Möhlin (CH); Schadt, Martin, CH-4411 Seltisberg (CH)

(56) Entgegenhaltungen:
- EP-A- 0 090 282
- EP-A- 0 315 050
- EP-A- 0 458 176
- EP-A- 0 475 273
- EP-A- 0 546 298
- WO-A-91/15450

## Beschreibung

Die vorliegende Erfindung betrifft neue Verbindungen mit einem Cyclohexylalkenoat Rest, flüssigkristalline Gemische, die solche Verbindungen enthalten, sowie die Verwendung solcher Verbindungen bzw. Gemische für elektro-optische Zwecke.

Flüssige Kristalle werden vor allem als Dielektrika in Anzeigevorrichtungen verwendet, da die optischen Eigenschaften solcher Substanzen durch eine angelegte Spannung beeinflusst werden können. Elektro-optische Vorrichtungen auf der Basis von Flüssigkristallen sind dem Fachmann bestens bekannt und können auf verschiedenen Effekten beruhen. Derartige Vorrichtungen sind beispielsweise Zellen mit dynamischer Streuung, DAP-Zellen (Deformation aufgerichteter Phasen), Gast/Wirt-Zellen, TN-Zellen mit verdrillt nematischer ("twisted nematic") Struktur, STN-Zellen ("super twisted nematic"), SBE-Zellen ("super birefringence effect") und OMI-Zellen ("optical mode interference"). Die gebräuchlichsten Anzeigevorrichtungen beruhen auf dem Schadt-Helferich-Effekt und besitzen eine verdrillt nematische Struktur.

Die Flüssigkristallmaterialien sollten eine gute chemische und thermische Stabilität haben und ausserdem gegenüber elektrischen Feldern und elektromagnetischen Strahlungen stabil sein. Sie sollten bei betriebsüblichen Temperaturen eine geeignete Mesophase besitzen, beispielsweise eine nematische, cholesterische oder getiltete smektische Phase. Die Flüssigkristallmaterialien sollten ferner niedere Viskosität aufweisen und in den Zellen kurze Ansprechzeiten, tiefe Schwellenspannungen und einen hohen Kontrast ergeben.

Weitere Eigenschaften, wie die elektrische Leitfähigkeit, die dielektrische Anisotropie und die optische Anisotropie, müssen je nach Zellentyp und Anwendungsgebiet unterschiedlichen Anforderungen genügen. Beispielsweise sollten Materialien für Zellen mit verdrillt nematischer Struktur eine positive dielektrische Anisotropie und eine möglichst geringe elektrische Leitfähigkeit aufweisen. Neben dem generellen Interesse an Flüssigkristallmaterialien mit hoher optischer Anisotropie besteht in letzter Zeit ein vermehrtes Interesse an Materialien mit niedriger optischer Anisotropie, insbesondere für aktiv adressierte Flüssigkristellanzeigen, z.B. bei TFT-Anwendungen ("thin film transistor") in Fernsehgeräten.

Flüssigkristalle werden zwecks Optimierung der Eigenschalten in der Begel als Mischungen mehrerer Komponenten verwendet. Es ist daher auch wichtig, dass die Komponenten untereinander gut mischbar sind.

Derartige Verbindungen werden nun durch die vorliegende Erfindung zur Verfügung gestellt.

Gegenstand der vorliegenden Erfindung sind Verbindungen der allgemeinen Formel 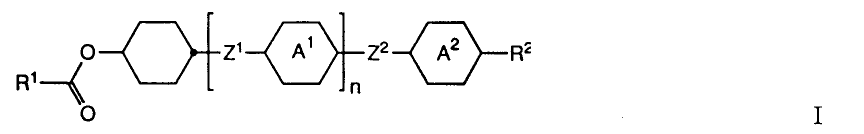 worin
- R¹: eine 1E-Alkenylgruppe mit 3 bis 7 Kohlensoffatomen bezeichnet;
- A¹ und A²: unabhängig voneinander unsubstituiertes oder mit Halogen substituiertes 1,4-Phenylen, in welchem falls unsubstituiert gegebenenfalls 1 oder 2 CH-Gruppen durch Stickstoff ersetzt sind, oder trans-1,4-Cyclohexylen oder trans-1,3-Dioxan-2,5-diyl darstellen;
- Z¹ und Z²: unabhängig voneinander eine einfache Kovalenzbindung,
-CH₂CH₂-, -COO-, -OOC-, -OCH₂-, -CH₂O-, -C≡C-, -(CH₂)₄-, -O(CH₂)₃-, -(CH₂)₃O- oder die trans-Form von -OCH₂CH=CH-, -CH=CHCH₂O-, -(CH₂)₂CH=CH- oder -CH=CH(CH₂)₂-bedeuten;
- n: entweder 0, 1 oder 2 ist; und
- R²: Alkyl, Alkoxy, Alkenyl oder Alkenyloxy mit 1 bzw. 2 bis 7 Kohlenstoffatomen oder,
in dem Fall, daß A¹ oder A² ein unsubstituierter oder mit Fluor substituiertes 1,4-Phenylen, Pyrimidin-2,5-diyl oder Pyridin-2,5-diyl bedeutet, auch Fluor, Chlor oder Cyano darstellt.

Es wurde gefinden, dass die Einführung einer Alkenylcarboxylgruppe die Tendenz zur Ausbildung von Flüssigkristallphasen, vor allem die Tendenz zur Ausbildung einer nematischen Phase günstig beeinflusst. Diese Ester führen in nematischen Mischungen zu hohen Klärpunkten und zu überraschend kurzen Schaltzeiten, dabei werden unerwünschte smektische Phasen wie beispielsweise S_{B}-Phasen oft unterdrückt. Die dielektrische Anisotropie (Δε) und die optische Anisotropie (Δn) können je nach Wahl der Ringe und Substituenten variiert werden. So haben beispielsweise Verbindungen der Formel I, worin R² Cyano und Z²-COO- bedeutet, eine hohe positive dielektrische Anisotropie. Verbindungen der Formel I, worin die Ringe A¹ und A² 1,4-Phenylen, in welchem gegebenenfalls eine oder zwei CH-Gruppen durch Stickstoff ersetzt sind, haben beispielsweise eine hohe optische Anisotropie.

Ganz besonders bevorzugte Reste R¹ haben 3 bis 7 Kohlenstoffatome; solche Reste sind beispielsweise 1E-Propenyl, 1E-Butenyl, 1E-Pentenyl, 1E-Hexenyl, 1E-Heptenyl und dergleichen.

Der Ausdruck "unsubstituiertes oder mit Halogen substituiertes 1,4-Phenylen, in welchem gegebenenfalls 1 oder 2 CH-Gruppen durch Stickstoff ersetzt sind" umfasst im Rahmen der vorliegenden Erfindung Gruppen wie 1,4-Phenylen, 2-Fluor-1,4-phenylen (dh. in ortho-Stellung zum Rest R²), 2,3-Difluor-1,4-phenylen, 2,6-Difluor-1,4-phenylen, 2-Chlor-1,4-phenylen, 2-Brom-1,4-phenylen, Pyridin-2,5-diyl, Pyrimidin-2,5-diyl und dergleichen. Bevorzugte Gruppen sind 1,4-Phenylen, 2-Fluor-1,4-phenylen, 2,3-Difluor-1,4-phenylen, 2,6-Difluor-1,4-phenylen, Pyridin-2,5-diyl, Pyrimidin-2,5-diyl.

Der Ausdruck "Halogen" bezeichnet Chlor, Fluor, Brom und Jod, insbesondere Chlor und Fluor.

R² ist bevorzugt Alkyl, 1E-Alkenyl, 3E-Alkenyl, Alkenyl mit endständiger Doppelbindung, Alkoxy, 2E-Alkenyloxy, 3-Alkenyloxy, Alkenyloxy mit endständiger Doppelbindung, und dergleichen. Beispiele bevorzugter Reste sind Methyl, Aethyl, Propyl, Butyl, Pentyl, Hexyl, Heptyl, Vinyl, 1E-Propenyl, 1E-Butenyl, 1E-Pentenyl, 1E-Hexenyl, 1E-Heptenyl, 3-Butenyl, 3E-Pentenyl, 3E-Hexenyl, 3E-Heptenyl, 4-Pentenyl, 5-Hexenyl, 6-Heptenyl Methoxy, Aethoxy, Propyloxy, Butyloxy, Pentyloxy, Hexyloxy, Heptyloxy, Allyloxy, 2E-Butenyloxy, 2E-Pentenyloxy, 2E-Hexenyloxy, 2E-Heptenyloxy, 3-Butenyloxy, 3Z-Pentenyloxy, 3Z-Hexenyloxy, 3Z-Heptenyloxy, 4-Pentenyloxy, 5-Hexenyloxy, 6-Heptenyloxy, und dergleichen. Besonders bevorzugte Reste R² haben 1, bzw. 2 bis 7 Kohlenstoffatome.

Bevorzugte Verbindungen der Formel I sind diejenigen Verbindungen, worin n = 0 oder 1 ist, insbesondere die Verbindungen der allgemeinen Formeln 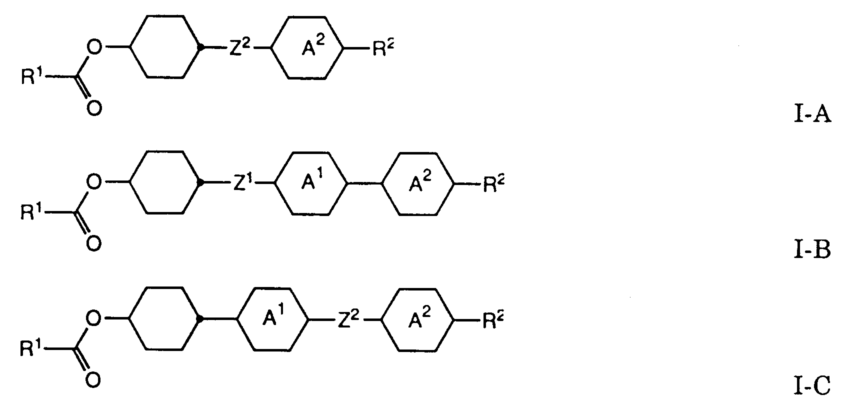 worin
- R¹: 1E-Alkenyl mit 3 bis 7 Kohlenstoffatomen bedeutet,
- A¹: unsubstituiertes oder mit Fluor substituiertes 1,4-Phenylen, oder trans-1,4-Cyclohexylen bedeutet,
- A²: unsubstituiertes oder mit Fluor substituiertes 1,4-Phenylen, Pyrimidin-2,5-diyl, Pyridin-2,5-diyl oder trans-1,4-Cyclohexylen bedeutet,
- Z¹, Z²: unabhängig voneinander eine einfache Kovalenzbindung, -CH₂CH₂-, -CH₂O-, -OCH₂-, -COO- oder -OOC- darstellen, und
- R²: Alkyl, Alkoxy, Alkenyl oder Alkenyloxy mit 1 bzw. 2 bis 7 Kohlenstoffatomen oder,
in dem Fall daß A¹ oder A² ein unsubstituierter oder mit Fluor substituiertes 1,4-Phenylen, Pyrimidin-2,5-diyl oder Pyridin-2,5-diyl bedeutet, auch Fluor, Chlor oder Cyano darstellt.

Ein bevorzugter Aspekt der Erfindung betrifft Verbindungen der Formeln I, I-A, I-B und I-C, worin R¹ 1E-Propenyl oder 1E-Butenyl darstellt, und R² Alkyl oder Alkoxy mit 1 bis 7 Kohlenstoffatomen bedeutet. In den Verbindungen der allgemeinen Formeln I, I-A, I-B und I-C steht vorzugsweise eine der Gruppen Z¹ und Z² für eine einfache Kovalenzbindung und die andere der Gruppen Z¹ und Z² für eine einfache Kovalerzbindung, -CH₂CH₂-, -COO- oder -OOC-.

Ein ganz besonders bevorzugter Aspekt dieser Erfindung betrifft die Verbindungen der allgemeinen Formeln 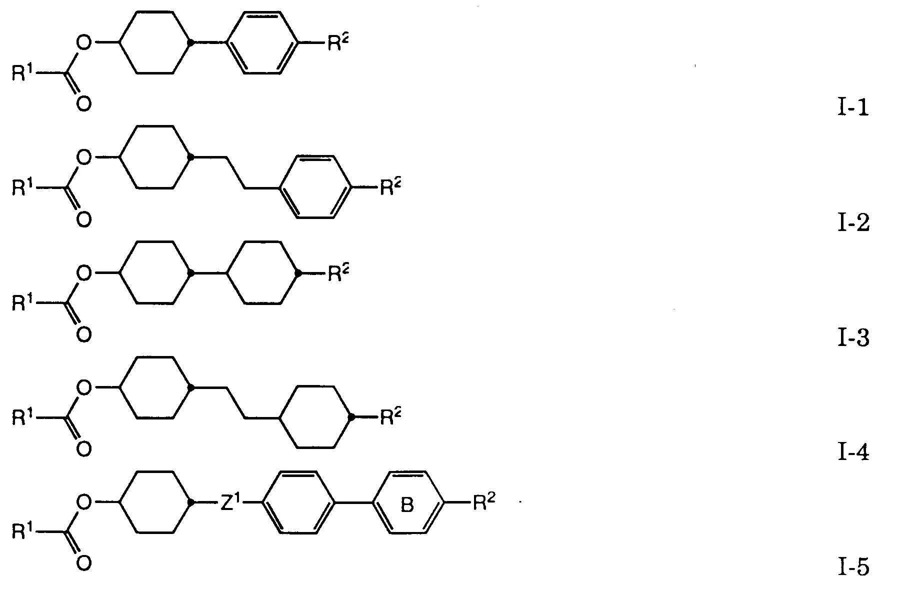 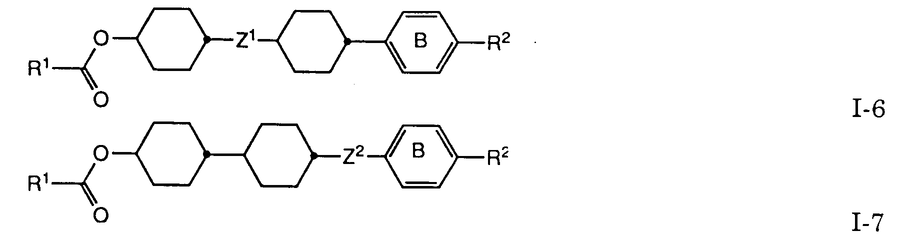 worin
- R¹: 1E-Propenyl oder 1E-Butenyl darstellt,
- Z¹, Z²: eine einfache Kovalenzbindung, -CH₂CH₂-, -COO- oder -OOC-bedeuten;
- Ring B: 1,4-Phenylen, 2-Fluor-1,4-phenylen (dh. in ortho-Stellung zum Rest R²), 2,3-Difluor-1,4-phenylen, 2,6-Difluor-1,4-phenylen darstellt, und
- R²: in den Verbindungen I-1 und I-2 Alkyl oder Alkoxy mit 1 bis 7 Kohlenstoffatomen oder Cyano,
in den Verbindungen I-3 und I-4 Alkyl oder Alkoxy mit 1 bis 7 Kohlenstoffatomen und
in den Verbindungen I-5, I-6 und I-7 Cyano bedeutet.

In den Verbindungen der Formel I-A, insbesondere in den Verbindungen der Formeln I-1 bis I-4, bedeutet R² vorzugsweise Alkyl oder Alkoxy mit 1 bis 5 Kohlenstoffatomen. Diese Verbindungen zeichnen sich durch eine überraschend breite nematische Mesophase und durch eine niedrige Viskosität aus.

Die Verbindungen der Formeln I-B und I-C, und insbesondere diejenigen der Formeln I-5 bis I-7, worin R² Fluor, Chlor oder Cyano bedeutet, und Z¹ bzw. Z² eine einfache Kovalenzbindung oder -CH₂CH₂- bedeutet, eignen sich dank ihrer niedrigen Schwellenspannung ganz besonders für die Anwendung in Flüssigkristallmaterialien, welche für TFT-Zellen verwendet werden.

Die vorliegende Erfindung bietet somit eine breite Palette neuer Komponenten zur weiteren Optimierung und Modifizierung von Flüssigkristallmaterialien.

In der EP-A-0 090 282 werden Polymere mit mesogenen Gruppen in den Seitenketten und unter anderem auch mesogene Gruppen mit einem Acrylatrest offenbart. Die Acrylatgruppe dient dazu, die mesogene Gruppen in das Polymer einzubinden.

In WO-A-91 15450 werden Alkenyl-COO-Reste gernerisch mitumfasst, jedoch wird kein Hinweis darauf gegeben, wie sich ein 1E-Alkenyl-COO-Rest auf die Eigenschaften einer Verbindung auswirkt.

In EP-A-0 458 176, EP-A-0 475 273 und EP-A-0 315 050 werden Verbindungen beschrieben, die anstatt eines Alkenyl-COO-Restes der vorliegenden Erfindung einen Alkenylrest aufweisen.

Die Verbindungen der allgemeinen Formel I können auf an sich bekannte Weise, wie beispielsweise nach der im Schema sowie in den Beispielen illustrierten Methode, hergestellt werden. Die Verbindungen der Formel II sind bekannte oder Analoge bekannter Verbindungen; solche Verbindungen sind beispielsweise in EP 315 014 oder EP 315 050 beschrieben. 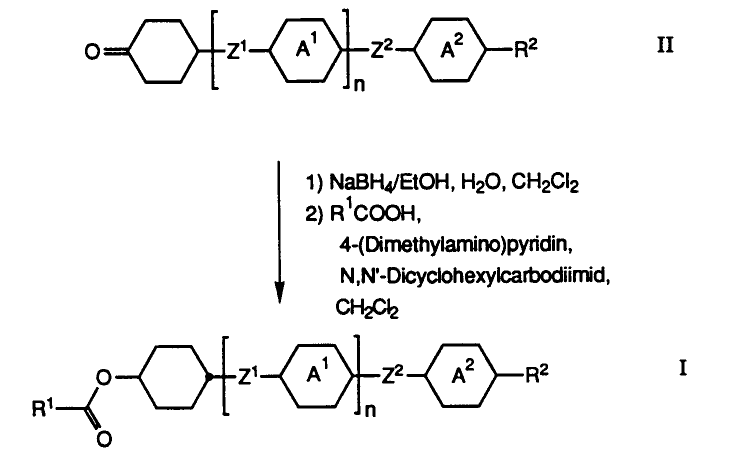 worin R¹, R², Z¹, Z², A¹, A² und n die in Formel I definierten Bedeutungen haben.

Die Verbindungen der Formel I können in Form von Gemischen untereinander und/oder mit anderen Flüssigkristallkomponenten verwendet werden.

Die Erfindung betrifft daher ebenfalls flüssigkristalline Gemische mit mindestens 2 Komponenten, welche dadurch gekennzeichnet sind, dass mindestens eine Komponente eine Verbindung der Formel I ist. Eine zweite und gegebenenfalls weitere Komponten können weitere Verbindungen der allgemeinen Formel I oder andere geeignete Flüssigkristallkomponenten sein. Geeignete Flüssigkristallkomponenten sind dem Fachmann in grosser Zahl bekannt, z.B. aus D. Demus et al., Flüssige Kristalle in Tabellen, VEB Deutscher Verlag für Grundstoffindustrie, Leipzig, Bände I und II, und viele davon sind zudem im Handel erhältlich.

Aufgrund der guten Löslichkeit der Verbindungen der Formel I in anderen Flüssigkristallmaterialien und aufgrund ihrer guten Mischbarkeit untereinander kann der Anteil an Verbindungen der Formel I in den erfindungsgemässen Gemischen relativ hoch sein und beispielsweise 1-70 Gew.-% betragen. Im allgemeinen ist ein Anteil von etwa 3-40 Gew.-%, insbesondere von etwa 5-30 Gew.% an Verbindungen der Formel I bevorzugt.

Vorzugsweise enthalten die erfindungsgemässen Gemische neben einer oder mehreren Verbindungen der Formel I eine oder mehrere Verbindungen aus der Gruppe der Verbindungen der allgemeinen Formeln 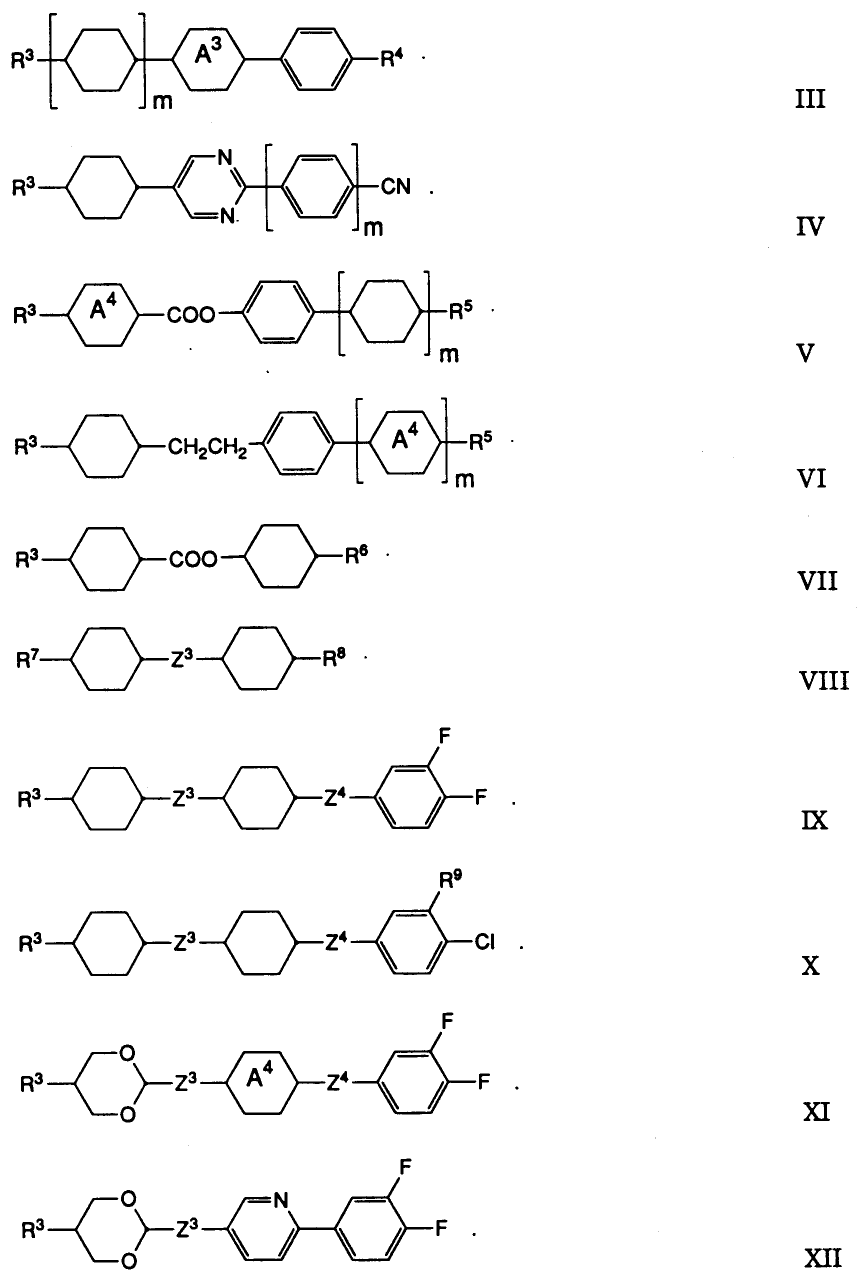 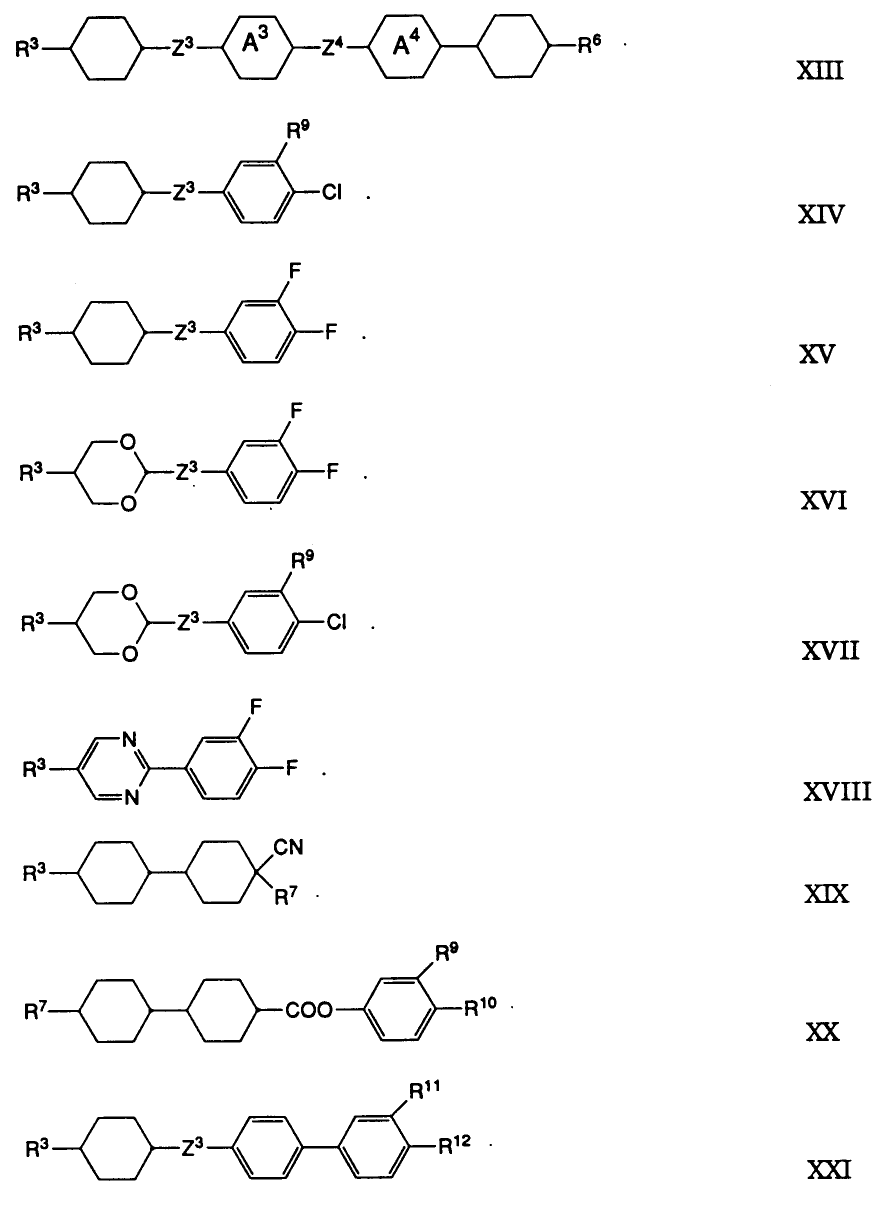 worin
- R³,R⁶: Alkyl, Alkoxyalkyl, 3E-Alkenyl, 4-Alkenyl oder an gesättigten Ringen auch 1E-Alkenyl bedeutet;
- m: 0 oder 1 bedeutet;
- Ring A³: 1,4-Phenylen, Pyridin-2,5-diyl, Pyrimidin-2,5-diyl, trans-1,4-Cyclohexylen oder trans-1,3-Dioxan-2,5-diyl bezeichnet;
- R⁴: Cyano, Isothiocyanato, Fluor, Alkyl, 3E-Alkenyl, 4-Alkenyl, Alkoxy, 2E-Alkenyloxy, 3-Alkenyloxy oder 1-Alkinyl darstellt;
- Ring A⁴: 1,4-Phenylen oder trans-1,4-Cyclohexylen bedeutet;
- R⁵: Alkyl, 3E-Alkenyl, 4-Alkenyl, oder an trans-1,4-Cyclohexylen auch 1E-Alkenyl, oder an 1,4-Phenylen auch Cyano, Isothiocyanato, Alkoxy, 2E-Alkenyloxy oder 3-Alkenyloxy bezeichnet;
- R⁷: Alkyl, 1E-Alkenyl, 3E-Alkenyl oder 4-Alkenyl bedeutet;
- R⁸: Cyano, Alkyl, 1E-Alkenyl, 3E-Alkenyl, 4-Alkenyl, Alkoxy, 2E-Alkenyloxy, 3-Alkenyloxy, Alkoxymethyl oder (2E-Alkenyl)oxymethyl darstellt;
- Z³, Z⁴: eine einfache Kovalenzbindung oder -CH₂CH₂- bezeichnen, wobei zwei aromatische Ringe immer durch eine einfache Kovalenzbindung verknüpft sind;
- R⁹: Wasserstoff, Fluor oder Chlor bedeutet;
- R¹⁰: Cyano, Fluor oder Chlor darstellt;
- R¹¹: Wasserstoff oder Fluor bezeichnet;
- R¹²: Fluor oder Chlor darstellt.

Der obenerwähnte Ausdruck " aromatische Ringe bezeichnet in diesem Zusammenhang Ringe wie beispielsweise 1,4-Phenylen, Pyridin-2,5-diyl oder Pyrimidin-2,5-diyl. Der Ausdruck "gesättigte Ringe" bezeichnet trans-1,4-Cyclohexylen oder 1,3-Dioxan-2,5-diyl.

Reste R³ bis R⁸ besitzen vorzugsweise je 1 bzw. 2 bis 12 Kohlenstoffatome, besonders bevorzugt je 1 bzw. 2 bis 7 Kohlenstoffatome. Geradkettige Reste sind im allgemeinen bevorzugt. Der Ausdruck "Alkyl" bedeutet in diesem Zusammenhang vorzugsweise geradkettige Reste mit 1 bis 12 Kohlenstoffatomen, vorzugsweise mit 1 bis 7 Kohlenstoffatomen wie beispielsweise Methyl, Aethyl, Propyl, Butyl, Pentyl, Hexyl oder Heptyl.

Der Ausdruck "Alkyloxyalkyl" bedeutet in diesem Zusammenhang vorzugsweise geradkettige Reste wie beispielsweise Methoxymethyl, Aethoxymethyl, Propyloxymethyl, Butyloxymethyl und dergleichen.

Der Ausdruck "Alkyloxy" bedeutet in diesem Zusammenhang vorzugsweise geradkettige Reste wie beispielsweise Methoxy, Aethoxy, Propyloxy, Butyloxy, Pentyloxy, Hexyloxy, Heptyloxy und dergleichen.

Der Ausdruck "1E-Alkenyl" bedeutet in diesem Zusammenhang vorzugsweise geradkettige Alkenylreste, in denen die Doppelbindung sich in 1-Stellung befindet, wie beispielsweise Vinyl, 1E-Propenyl, 1E-Butenyl, 1E-Pentenyl, 1E-Hexenyl, 1E-Heptenyl und dergleichen.

Der Ausdruck "3E-Alkenyl" bedeutet in diesem Zusammenhang vorzugsweise geradkettige Alkenylreste, in denen die Doppelbindung sich in 3-Stellung befindet, wie beispielsweise 3-Butenyl, 3E-Pentenyl, 3E-Hexenyl, 3E-Heptenyl und dergleichen.

Der Ausdruck "4-Alkenyl" bedeutet in diesem Zusammenhang vorzugsweise geradkettige Alkenylreste, in denen die Doppelbindung sich in 4-Stellung befindet, wie beispielsweise 4-Pentenyl, 4-Hexenyl, 4-Heptenyl und dergleichen.

Der Ausdruck "2E- bzw. 3-Alkenyloxy" bedeutet in diesem Zusammenhang vorzugsweise geradkettige Alkenyloxyreste, in denen die Doppelbindung sich in 2- bzw 3-Stellung befindet und E bzw. Z die bevorzugte Konfiguration angibt, wie beispielsweise Allyloxy, 2E-Butenyloxy, 2E-Pentenyloxy, 2E-Hexenyloxy, 2E-Heptenyloxy, 3-Butenyloxy, 3-Pentenyloxy, 3-Hexenyloxy, 3-Heptenyloxy, 4-Pentenyloxy, 5-Hexenyloxy, 6-Heptenyloxy und dergleichen.

Der Ausdruck "1-Alkinyl" bedeutet in diesem Zusammenhang vorzugsweise geradkettige Alkinylreste, in denen die Dreifachbindung sich in 1-Stellung befindet, wie beispielsweise Aethinyl, 1-Propinyl, 1-Butinyl, 1-Pentinyl und dergleichen.

Die erfindungsgemässen Mischungen können ferner optisch aktive Verbindungen (z.B. optisch aktive 4'-Alkyl. oder 4'-Alkoxy-4-biphenylcarbonitrile) und/oder dichroitische Farbstoffe (z.B. Azo-, Azoxy- oder Anthrachinonfarbstoffe) enthalten. Der Anteil solcher Verbindungen wird durch die Löslichkeit, die gewünschte Ganghöhe, Farbe, Extinktion und dergleichen bestimmt. Im allgemeinen beträgt der Anteil optisch aktiver Verbindungen und dichroitischer Farbstoffe höchstens je etwa 10 Gew.-% im Gesamtgemisch.

Die Herstellung der erfindungsgemässen Gemische und die Herstellung der elektro-optischen Vorrichtungen können in an sich bekannter Weise erfolgen.

Die Herstellung der Verbindungen der Formel I sowie flüssigkristalline Gemische enthaltend diese Verbindungen werden durch die folgenden Beispiele weiter veranschaulicht. C bedeutet eine kristalline, S eine smektische, N eine nematische und I die isotrope Phase. V₁₀ bezeichnet die Spannung für 10% Transmission (Blickrichtung senkrecht zur Plattenoberfläche). tₒₙ und t_{off} bezeichnen die Einschaltzeit bzw. die Ausschaltzeit und Δn bezeichnet die optische Anisotropie.

### Beispiel 1

Zu einer Lösung von 5,0 g trans-4-(trans-4-[3,4-difluorphenyl]cyclohexyl)cyclohexan-1-ol, 1,5 g Crotonsäure und 0,05 g 4-(Dimethylamino)pyridin in 50 ml Dichlormethan wird unter Rühren innert 5 Minuten bei 0°C 4,2 g N,N '-Dicyclohexylcarbodiimid gegeben. Das Reaktionsgemisch wird über Nacht bei Raumtemperatur weitergerührt, dann filtriert, das Filtrat wird mit gesättigter Natriumbicarbonat-Lösung und mit Wasser gewaschen und eingeengt. Chromatographische Reinigung des Rückstandes an Kieselgel mit Hexan/Aethylacetat (Vol. 9:1) und zweimaliges Umkristallisieren der gemäss Dünnschichtchromatographie reinen Fraktionen ergibt 2,0 g trans-4-(trans-4-[3,4-Difluorphenyl]cyclohexyl)cyclohexyl-(E)-2-butenoat. Smp. (C-N) 103°C, und Klp. (N-I) 201°C.

Das als Ausgangsmaterial verwendete trans-4-(trans-4-[3,4-difluorphenyl]cyclohexyl)cyclohexan-1-ol wird wie folgt hergestellt:

Ein Gemisch von 32 g Natriumborhydrid und 180 ml Aethanol wird bei 0°C mit 165 ml Wasser versetzt, 10 Minuten weitergerührt, dann tropfenweise bei 0-5°C mit einer Lösung von 50 g trans-4-(trans-4-[3,4-difluorphenyl]cyclohexyl)cyclohexanon in 100 ml Aethanol und 10 ml Dichlormethan versetzt. Das Reaktionsgemisch wird weitere 30 Minuten bei 0°C gerührt, dann auf 100 ml Dichlormethan gegossen und zweimal mit je 100 ml Wasser gewaschen. Die vereinigten wässrigen Phasen werden zweimal mit je 50 ml Dichlormethan extrahiert. Die vereinigten organischen Phasen werden dann zweimal mit je 100 mi Wasser gewaschen, über Magnesiumsulfat getrocknet, die Suspension filtriert und das Filtrat eingeengt. Der Rückstand wird aus tert. Butylmethyläther umkristallisiert. Dies ergibt 35 g trans-4-(trans-4-[3,4-difluorphenyl]cyclohexyl)cyclohexan-1-ol, Smp. (C-I) 144-145°C.

Auf analoge Weise können folgende Verbindungen hergestellt werden:
trans-4-(trans-4-[3,4-Difluorphenyl]cyclohexyl)cyclohexyl-(E)-2-pentenoat.
trans-4-(trans-4-[4-Fluorphenyl]cyclohexyl)cyclohexyl-(E)-2-butenoat, Smp. (C-N) 105°C, Klp. (N-I) 219°C.
trans-4-(trans-4-[4-Fluorphenyl]cyclohexyl)cyclohexyl-(E)-2-pentenoat.
trans-4-(trans-4-[4-Chlorphenyl]cyclohexyl)cyclohexyl-(E)-2-butenoat, Smp. (C-N) 129°C, Klp. (N-I) 257°C.
trans-4-(trans-4-[4-Chlorphenyl]cyclohexyl)cyclohexyl-(E)-2-pentenoat.
trans-4-(trans-4-[4-Chlor-3-fluorphenyl]cyclohexyl)cyclohexyl-(E)-2-butenoat, Smp. (C-N) 134°C, Klp. (N-I) 233°C.
trans-4-(trans-4-[4-Chlor-3-fluorphenyl]cyclohexyl)cyclohexyl-(E)-2-pentenoat.
trans-4-(trans-4-[4-Cyanophenyl]cyclohexyl)cyclohexyl-(E)-2-butenoat.
trans-4-(trans-4-[4-Cyanophenyl]cyclohexyl)cyclohexyl-(E)-2-pentenoat.
trans-4-(trans-4-[4-Cyano-3-fluorphenyl]cyclohexyl)cyclohexyl-(E)-2-butenoat.
trans-4-(trans-4-[4-Cyano-3-fluorphenyl]cyclohexyl)cyclohexyl-(E)-2-pentenoat.
trans-4-(trans-4-[4-Methylphenyl]cyclohexyl)cyclohexyl-(E)-2-butenoat.
trans-4-(trans-4-[4-Methylphenyl]cyclohexyl)cyclohexyl-(E)-2-pentenoat.
trans-4-(trans-4-[4-Aethylphenyl]cyclohexyl)cyclohexyl-(E)-2-butenoat.
trans-4-(trans-4-[4-Aethylphenyl]cyclohexyl)cyclohexyl-(E)-2-pentenoat.
trans-4-(trans-4-[4-Propylphenyl]cyclohexyl)cyclohexyl-(E)-2-butenoat.
trans-4-(trans-4-[4-Propylphenyl]cyclohexyl)cyclohexyl-(E)-2-pentenoat.
trans-4-(trans-4-[4-Methoxyphenyl]cyclohexyl)cyclohexyl-(E)-2-butenoat.
trans-4-(trans-4-[4-Methoxyphenyl]cyclohexyl)cyclohexyl-(E)-2-pentenoat.
trans-4-(trans-4-[4-Aethoxyphenyl]cyclohexyl)cyclohexyl-(E)-2-butenoat.
trans-4-(trans-4-[4-Aethoxyphenyl]cyclohexyl)cyclohexyl-(E)-2-pentenoat.
trans-4-(trans-4-[2-(4-Fluorphenyl)äthyl]cyclohexyl)cyclohexyl-(E)-2-butenoat.
trans-4-(trans-4-[2-(4-Chlorphenyl)äthyl]cyclohexyl)cyclohexyl-(E)-2-butenoat.
trans-4-(trans-4-[2-(4-Cyanophenyl)äthyl]cyclohexyl)cyclohexyl-(E)-2-butenoat.
trans-4-(trans-4-[2-(3,4-Difluorphenyl)äthyl]cyclohexyl)cyclohexyl-(E)-2-butenoat.
trans-4-(trans-4-[2-(4-Chlor-3-fluorphenyl)äthyl]cyclohexyl)cyclohexyl-2-(E)-butenoat.
trans-4-[3,4-Difluorphenyl]cyclohexyl-(E)-2-butenoat.
trans-4-[3,4-Difluorphenyl]cyclohexyl-(E)-2-pentenoat.
trans-4-[4-Fluorphenyl]cyclohexyl-(E)-2-butenoat.
trans-4-[4-Fluorphenyl]cyclohexyl-(E)-2-pentenoat.
trans-4-[4-Chlorphenyl]cyclohexyl-(E)-2-butenoat.
trans-4-[4-Chlorphenyl]cyclohexyl-(E)-2-pentenoat.
trans-4-[4-Chlor-3-fluorphenyl]cyclohexyl-(E)-2-butenoat.
trans-4-[4-Chlor-3-fluorphenyl]cyclohexyl-(E)-2-pentenoat.
trans-4-[4-Cyanophenyl]cyclohexyl-(E)-2-butenoat, Smp. (C-I) 128°C, Klp. (N-I) 119°C.
trans-4-[4-Cyanophenyl]cyclohexyl-(E)-2-pentenoat.
trans-4-[4-Cyano-3-fluorphenyl]cyclohexyl-(E)-2-butenoat.
trans-4-[4-Cyano-3-fluorphenyl]cyclohexyl-(E)-2-pentenoat.
trans-4-[4-Methylphenyl]cyclohexyl-(E)-2-butenoat.
trans-4-[4-Methylphenyl]cyclohexyl-(E)-2-pentenoat.
trans-4-[4-Aethylphenyl]cyclohexyl-(E)-2-butenoat.
trans-4-[4-Aethylphenyl]cyclohexyl-(E)-2-pentenoat
trans-4-[4-Propylphenyl]cyclohexyl-(E)-2-butenoat, Smp. (C-I) 71°C, Klp. (N-I) 47°C.
trans-4-4-[4-Propylphenyl]cyclohexyl-(E)-2-pentenoat.
trans-4-[4-Methoxyphenyl]cyclohexyl-(E)-2-butenoat, Smp. (C-N) 74°C, Klp. (N-I) 89°C.
trans-4-[4-Methoxyphenyl]cyclohexyl-(E)-2-pentenoat.
trans-4-[4-Aethoxyphenyl]cyclohexyl-(E)-2-butenoat, Smp. (C-N) 78°C, Klp. (N-I) 110°C.
trans-4-[4-Aethoxyphenyl]cyclohexyl-(E)-2-pentenoat, Smp. (C-I) 84°C, Klp. (N-I) 80°C.
trans-4-[4-Aethoxyphenyl]cyclohexyl-(E)-2-hexenoat, Smp. (C-N) 53°C, Klp. (N-I) 86°C.
trans-4-[4-Aethoxyphenyl]cyclohexyl-(E)-2-heptenoat, Smp. (C-N) 46°C, Klp. (N-I) 68°C.
trans-4-[4-Propyloxyphenyl]cyclohexyl-(E)-2-butenoat, Smp. (C-N) 65°C, Klp. (N-I) 87°C.
trans-4-[4-Butoxyphenyl]cyclohexyl-(E)-2-butenoat, Smp. (C-N) 71°C, Klp. (N-I) 92°C.
trans-4-[4-Pentyloxyphenyl]cyclohexyl-(E)-2-butenoat, Smp. (C-N) 75°C, Klp. (N-I) 79°C.
trans-4-[4-Hexyloxyphenyl]cyclohexyl-(E)-2-butenoat, Smp. (C-N) 67°C, Klp. (N-I) 80°C.
trans-4-[4-Heptyloxyphenyl]cyclohexyl-(E)-2-butenoat, Smp. (C-N) 61°C, Klp. (N-I) 74°C.
trans-4-[2-(4-Fluorphenyl)äthyl]cyclohexyl-(E)-2-butenoat, Smp (C-I) 62°C.
trans-4-[2-(4-Chlorphenyl)äthyl]cyclohexyl-(E)-2-butenoat, Smp (C-I) 73°C, Klp. (N-I) 45°C.
trans-4-[2-(4-Cyanophenyl)äthyl]cyclohexyl-(E)-2-butenoat.
trans-4-[2-(3,4-Difluorphenyl)äthyl]cyclohexyl-(E)-2-butenoat.
trans-4-[2-(4-Chlor-3-fluorphenyl)äthyl]cyclohexyl-(E)-2-butenoat,
trans-4-(4-[3,4-Difluor-4'-biphenyl])cyclohexyl-(E)-2-butenoat, Smp. (C-N) 113°C, Klp. (N-I) 184°C.
trans-4-(4-[3,4-Difluor-4'-biphenyl])cyclohexyl-(E)-2-pentenoat.
trans-4-(4-[4-Fluor-4'-biphenyl])cyclohexyl-(E)-2-butenoat, Smp. (C-N) 122°C, Klp. (N-I) 216°C.
trans-4-(4-[4-Fluor-4'-biphenyl])cyclohexyl-(E)-2-pentenoat.
trans-4-(4-[4-Chlor-4'-biphenyl])cyclohexyl-(E)-2-butenoat.
trans-4-(4-[4-Chlor-4'-biphenyl])cyclohexyl-(E)-2-pentenoat.
trans-4-(t4-[4-Chlor-3-fluor-4'-biphenyl])cyclohexyl-(E)-2-butenoat.
trans-4-(4-[4-Chlor-3-fluor-4'-biphenyl])cyclohexyl-(E)-2-pentenoat.
trans-4-(4-[4-Cyano-4'-bipheny]cyclohexyl-(E)-2-butenoat, Smp. (C-N) 171°C, Klp. (N-I) ∼ 290°C.
trans-4-(4-[4-Cyano-4'-biphenyl])cyclohexyl-(E)-2-pentenoat.
trans-4-(4-[4-Cyano-3-fluor-4'-biphenyl])cyclohexyl-(E)-2-butenoat.
trans-4-(4-[4-Cyano-3-fluor-4'-biphenyl])cyclohexyl-(E)-2-pentenoat.
trans-4-(4-[4-Aethyl-4'-biphenyl)cyclohexyl-(E)-2-butenoat.
trans-4-(4-[4-Aethyl-4'-biphenyl)cyclohexyl-(E)-2-pentenoat.
trans-4-(4-[4-Propyl-4'-biphenyl)cyclohexyl-(E)-2-butenoat.
trans-4-(4-[4-Propyl-4'-biphenyl)cyclohexyl-(E)-2-pentenoat.
trans-4-(4-[4-Methoxy-4'-biphenyl)cyclohexyl-(E)-2-butenoat.
trans-4-(4-[4-Methoxy-4'-biphenyl)cyclohexyl-(E)-2-pentenoat.
trans-4-(4-[4-Aethoxy-4'-biphenylcyclohexyl-(E)-2-butenoat.
trans-4-(4-[4-Aethoxy-4'-biphenyl)cyclohexyl-(E)-2-pentenoat.
trans-4-(4-[2,3-Difluor-4-methoxy-4'-biphenyl)cyclohexyl-(E)-2-butenoat, Smp. (C-N) 155°C, Klp. (N-I) 234°C.
trans-4-(4-[2,3-Difluor-4-äthoxy-4'-biphenyl)cyclohexyl-(E)-2-butenoat, Smp. (C-N) 120°C, Klp. (N-I) 240°C.
trans-4-(4-[2,3-Difluor-4-propoxy-4'-biphenyl)cyclohexyl-(E)-2-butenoat, Smp. (C-N) 96°C, Klp. (N-I) 225°C.
trans-4-(4-[2,3-Difluor-4-butoxy-4'-biphenyl)cyclohexyl-(E)-2-butenoat, Smp. (C-N) 103°C, Klp. (N-I) 218°C.
trans-4-(4-[2,3-Difluor-4-pentyloxy-4'-biphenyl)cyclohexyl-(E)-2-butenoat, Smp. (C-N) 105°C, Klp. (N-I) 201°C.
trans-4-(4-[2,3-Difluor-4-hexyloxy-4'-biphenyl)cyclohexyl-(E)-2-butenoat, Smp. (C-N) 76°C, Klp. (N-I) 195°C.
trans-4-(4-[2,3-Difluor-4-heptyloxy-4'-biphenyl)cyclohexyl-(E)-2-butenoat, Smp. (C-N) 74°C, Klp. (N-I) 186°C.
trans-4-(trans-4-Cyanocyclohexyl)cyclohexyl-(E)-2-butenoat.
trans-4-(trans-4-Methylcyclohexyl)cyclohexyl-(E)-2-butenoat.
trans-4-(trans-4-Methylcyclohexyl)cyclohexyl-(E)-2-pentenoat.
trans-4-(trans-4-Aethylcyclohexyl)cyclohexyl-(E)-2-butenoat.
trans-4-(trans-4-Aethylcyclohexyl)cyclohexyl-(E)-2-pentenoat.
trans-4-(trans-4-Propylcyclohexyl)cyclohexyl-(E)-2-butenoat, Smp. (C-N) 77°C, Klp. (N-I) 172°C.
trans-4-(trans-4-Propylcyclohexyl)cyclohexyl-(E)-2-pentenoat.
trans-4-(trans-4-Butylcyclohexyl)cyclohexyl-(E)-2-butenoat, Smp. (C-N) 67°C, Klp. (N-I) 122°C.
trans-4-(trans-4-Butylcyclohexyl)cyclohexyl-(E)-2-pentenoat.
trans-4-(trans-4-Pentylcyclohexyl)cyclohexyl-(E)-2-butenoat, Smp. (C-N) 74°C, Klp. (N-I) 131°C.
trans-4-(trans-4-Pentylcyclohexyl)cyclohexyl-(E)-2-pentenoat.
trans-4-[2-(trans-4-Methylcyclohexyl)äthyl]cyclohexyl-(E)-2-butenoat.
trans-4-[2-(trans-4-Methylcyclohexyl)äthyl]cyclohexyl-(E)-2-pentenoat.
trans-4-[2-(trans-4-Aethylcyclohexyl)äthyl]cyclohexyl-(E)-2-butenoat.
trans-4-[2-(trans-4-Aethylcyclohexyl)äthyl]cyclohexyl-(E)-2-pentenoat.
trans-4-[2-(trans-4-Propylcyclohexyl)äthyl]cyclohexyl-(E)-2-butenoat, Smp. (C-N) 44°C, Klp. (N-I) 109°C.
trans-4-[2-(trans-4-Propylcyclohexyl)äthyl]cyclohexyl-(E)-2-pentenoat, Smp. (C-S_{B}) 46°C, S_{B}-N, 62°C, Klp. (N-I) 86°C.
trans-4-[2-(trans-4-Propylcyclohexyl)äthyl]cyclohexyl-(E)-2-hexenoat, Smp. (C-S_{B}) 43°C, S_{B}-N, 69°C, Klp. (N-I) 89°C.
trans-4-[2-(trans-4-Propylcyclohexyl)äthyl]cyclohexyl-(E)-2-heptenoat, Smp. (C-S_{B}) 25°C, S_{B}-N, 71°C, Klp. (N-I) 78°C.
trans-4-[4-(5-Pentyl-2-pyrimidinyl)phenyl]cyclohexyl-(E)-2-butenoat
trans-4-[4-(5-Pentyl-2-pyrimidinyl)phenyl]cyclohexyl-(E)-2-pentenoat.
trans-4-[4-(5-Pentyl-2-pyrimidinyl)phenyl]cyclohexyl-(E)-2-hexenoat.
trans-4-[4-(5-Pentyl-2-pyrimidinyl)phenyl]cyclohexyl-(E)-2-heptenoat.
trans-4-[4-(5-Pentyl-2-pyridinyl)phenyl]cyclohexyl-(E)-2-butenoat
trans-4-[4-(5-Pentyl-2-pyridinyl)phenyl]cyclohexyl-(E)-2-pentenoat.
trans-4-[4-(5-Pentyl-2-pyridinyl)phenyl]cyclohexyl-(E)-2-hexenoat.
trans-4-[4-(5-Pentyl-2-pyridinyl)phenyl]cyclohexyl-(E)-2-heptenoat.

### Beispiel 2

0,7 g 4-Pentylphenol, 1,0 g trans-4-[(E)-2-Butenoyloxy)cyclohexancarbonsäure und 0,05 g 4-(Dimethylamino)pyridin und 50 ml Dichlormethan werden in analoger Weise zu Beispiel 1 zu 4-Pentylphenyl-trans-4-[(E)-2-butenoyloxy)cyclohexanoat umgesetzt.

Die als Ausgangsmaterial verwendete trans-4-[(E)-2-Butenoyloxy)cyclohexancarbonsäure wird wie folgt hergestellt:
b) Zu einer Lösung von 8,7 g 4-trans-Hydroxycyclohexancarbonsäure, 10 ml Triäthylamin und 100 ml Dichlormethan bei 0 °C wird unter Rühren innert 15 Minuten eine Lösung von Crotonsäurechlorid in 20 ml Dichlormethan zugetropft. Das Reaktionsgemisch wird 20 Minuten bei 0 °C und dann über Nacht bei Raumtemperatur weitergerührt, auf Wasser gegossen und dreimal mit je 50 ml Aethylacetat extrahiert. Die vereinigten organischen Phasen werden zweimal mit je 50 ml Wasser gewaschen, über Magnesiumsulfat getrocknet, filtriert und anschliessend eingeengt. Umkristallisieren aus Hexan ergibt 6,3 g trans-4-[(E)-2-Butenoyloxy)cyclohexancarbonsäure.

In analoger Weise können folgende Verbindungen hergestellt werden:
4-Methylphenyl-trans-4-[(E)-2-butenoyloxy]cyclohexanoat.
4-Aethylphenyl-trans-4-[(E)-2-butenoyloxy]cyclohexanoat.
4-Propylphenyl-trans-4-[(E)-2-butenoyloxy]cyclohexanoat.
4-Butylphenyl-trans-4-[(E)-2-butenoyloxy]cyclohexanoat.
4-Hexylphenyl-trans-4-[(E)-2-butenoyloxy]cyclohexanoat.
4-Heptylphenyl-trans-4-[(E)-2-butenoyloxy]cyclohexanoat.
4-Methoxyphenyl-trans-4-[(E)-2-butenoyloxy]cyclohexanoat.
4-Aethoxyphenyl-trans-4-[(E)-2-butenoyloxy]cyclohexanoat.
4-Propyloxyphenyl-trans-4-[(E)-2-butenoyloxy]cyclohexanoat.
4-Butyloxyphenyl-trans-4-[(E)-2-butenoyloxy]cyclohexanoat.
4-Pentyloxyphenyl-trans-4-[(E)-2-butenoyloxy]cyclohexanoat.
4-Hexyloxyphenyl-trans-4-[(E)-2-butenoyloxy]cyclohexanoat.
4-Fluorphenyl-trans-4-[(E)-2-butenoyloxy]cyclohexanoat.
4-Chlorphenyl-trans-4-[(E)-2-butenoyloxy]cyclohexanoat.
4-Cyanophenyl-trans-4-[(E)-2-butenoyloxy]cyclohexanoat.
3,4-Difluorphenyl-trans-4-[(E)-2-butenoyloxy]cyclohexanoat.
3-Fluor-4-chlorphenyl-trans-4-[(E)-2-butenoyloxy]cyclohexanoat.
3-Fluor-4-cyanophenyl-trans-4-[(E)-2-butenoyloxy]cyclohexanoat.
4-Methylphenyl-trans-4-(trans-4-[(E)-2-butenoyloxy]cyclohexyl)cyclohexanoat.
4-Aethylphenyl-trans-4-(trans-4-[(E)-2-butenoyloxy]cyclohexyl)cyclohexanoat.
4-Propylphenyl-trans-4-(trans-4-[(E)-2-butenoyloxy]cyclohexyl)cyclohexanoat.
4-Butylphenyl-trans-4-(trans-4-[(E)-2-butenoyloxy]cyclohexyl)cyclohexanoat.
4-Pentylphenyl-trans-4-(trans-4-[(E)-2-butenoyloxy]cyclohexyl)cyclohexanoat.
4-Hexylphenyl-trans-4-(trans-4-[(E)-2-butenoyloxy]cyclohexyl)cyclohexanoat.
4-Methoxyphenyl-trans-4-(trans-4-[(E)-2-butenoyloxy]cyclohexyl)cyclohexanoat.
4-Aethoxyphenyl-trans-4-(trans-4-[(E)-2-butenoyloxy]cyclohexyl)cyclohexanoat.
4-Propyloxyphenyl-trans-4-(trans-4-[(E)-2-butenoyloxy]cyclohexyl)cyclohexanoat.
4-Butyloxyphenyl-trans-4-(trans-4-[(E)-2-butenoyloxy]cyclohexyl)cyclohexanoat.
4-Pentyloxyphenyl-trans-4-(trans-4-[(E)-2-butenoyloxy]cyclohexyl)cyclohexanoat.
4-Hexyloxyphenyl-trans-4-(trans-4-[(E)-2-butenoyloxy]cyclohexyl)cyclohexanoat.
4-Fluorphenyl-trans-4-(trans-4-[(E)-2-butenoyloxy]cyclohexyl)cyclohexanoat.
4-Chlorphenyl-trans-4-(trans-4-[(E)-2-butenoyloxy]cyclohexyl)cyclohexanoat.
4-Cyanophenyl-trans-4-(trans-4-[(E)-2-butenoyloxy]cyclohexyl)cyclohexanoat.
3,4-Difluorphenyl-trans-4-(trans-4-[(E)-2-butenoyloxy]cyclohexyl)cyclohexanoat.
3-Fluor-4-chlorphenyl-trans-4-(trans-4-[(E)-2-butenoyloxy]cyclohexyl)cyclohexanoat.
3-Fluor-4-cyanophenyl-trans-4-(trans-4-[(E)-2-butenoyloxy]cyclohexyl)cyclohexanoat.
4-trans-Methylcyclohexyl-trans-4-[(E)-2-butenoyloxy]cyclohexanoat.
4-trans-Aethylcyclohexyl-trans-4-[(E)-2-butenoyloxy]cyclohexanoat.
4-trans-Propylcyclohexyl-trans-4-[(E)-2-butenoyloxy]cyclohexanoat.
4-trans-Butylcyclohexyl-trans-4-[(E)-2-butenoyloxy]cyclohexanoat.
4-trans-Pentylcyclohexyl-trans-4-[(E)-2-butenoyloxy]cyclohexanoat.
4-trans-Hexylcyclohexyl-trans-4-[(E)-2-butenoyloxy]cyclohexanoat.
4-trans-Heptylcyclohexyl-trans-4-[(E)-2-butenoyloxy]cyclohexanoat.
4-trans-Cyanocyclohexyl-trans-4-[(E)-2-butenoyloxy]cyclohexanoat.
4-trans-Methylcyclohexyl-trans-4-(trans-4-[(E)-2-butenoyloxy]cyclohexyl)cyclohexanoat.
4-trans-Aethylcyclohexyl-trans-4-(trans-4-[(E)-2-butenoyloxy]cyclohexyl)cyclohexanoat.
4-trans-Propylcyclohexyl-trans-4-(trans-4-[(E)-2-butenoyloxy]cyclohexyl)cyclohexanoat.
4-trans-Butylcyclohexyl-trans-4-(trans-4-[(E)-2-butenoyloxy]cyclohexyl)cyclohexanoat.
4-trans-Pentylcyclohexyl-trans-4-(trans-4-[(E)-2-butenoyloxy]cyclohexyl)cyclohexanoat.
4-trans-Hexylcyclohexyl-trans-4-(trans-4-[(E)-2-butenoyloxy]cyclohexyl)cyclohexanoat.
4-trans-Heptylcyclohexyl-trans-4-(trans-4-[(E)-2-butenoyloxy]cyclohexyl)cyclohexanoat.
4-trans-Cyanocyclohexyl-trans-4-(trans-4-[(E)-2-butenoyloxy]cyclohexyl)cyclohexanoat.

### Beispiel 3

Zur Untersuchung der Eigenschaften der Verbindungen der Formel I in Gemischen wurden binäre Mischungen (BM) mit 4-(trans-4-Pentylcyclohexyl)benzonitril hergestellt. Die Schwellenspannung wurden in einer TN-Zelle (low bias tilt) mit 8 µm Plattenabstand bei 22°C gemessen; als Betriebsspannung wurde der 2,5-fache Wert der Schwellenspannung (V₁₀) gewählt. Gemessen wurden der Klärpunkt (Klp. (N-I), die Schwellenspannung (V₁₀), die Einschaltzeit (tₒₙ), die Ausschaltzeit (t_{off}) sowie die optische Anisotropie (Δn). Die entsprechenden Daten für 4-(trans-4-Pentylcyclohexyl)benzonitril betragen: Klp. (N-I) 54,6°C, V₁₀ = 1,62V, tₒₙ = 22 ms, t_{off} = 40 ms und Δn = 0,120 .

### BM-1

- 90 Gew.%: 4-(trans-4-Pentylcyclohexyl)benzonitril
- 10 Gew.%: trans-4-(trans-4-[3,4-Difluorphenyl]cyclohexyl)cyclohexyl-(E)-2-butenoat.

Klp. (N-I) = 61,8 °C, V₁₀ = 1,66 V, Tₒₙ = 27 ms, T_{off} = 44 ms, Δn = 0,123.

### BM-2

- 80 Gew.%: 4-(trans-4-Pentylcyclohexyl)benzonitril
- 20 Gew.%: trans-4-(trans-4-[3,4-Difluorphenyl]cyclohexyl)cyclohexyl-(E)-2-butenoat.

Klp. (N-I) = 70,2 °C, V₁₀ = 1,70 V, Tₒₙ = 32 ms, T_{off} = 52 ms, Δn = 0,124

## Patentansprüche

1. Verbindungen der allgemeinen Formel 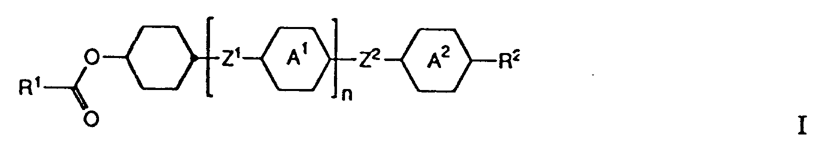 worin
R¹ eine 1E-Alkenylgruppe mit 3 bis 7 Kohlenstoffatomen bezeichnet;
A¹ und A² unabhängig voneinander unsubstituiertes oder mit Halogen substituiertes 1,4-Phenylen, in welchem falls unsubstituiert gegebenenfalls 1 oder 2 CH-Gruppen durch Stickstoff ersetzt sind, oder trans-1,4-Cyclohexylen oder trans-1,3-Dioxan-2,5-diyl darstellen;
Z¹ und Z² unabhängig voneinander eine einfache Kovalenzbindung,
-CH₂CH₂-, -COO-, -OOC-, -OCH₂-, -CH₂O-, -C≡C-, -(CH₂)₄-, -O(CH₂)₃-, -(CH₂)₃O- oder die trans-Form von -OCH₂CH=CH-, -CH=CHCH₂O-, -(CH₂)₂CH₂=CH- oder -CH=CH(CH₂)₂-bedeuten;
n entweder 0, 1 oder 2 ist; und
R² Alkyl, Alkoxy, Alkenyl oder Alkenyloxy mit 1 bzw. 2 bis 7 Kohlenstoffatomen oder,
in dem Fall, daß A¹ oder A² ein unsubstituierter oder mit Fluor substituiertes 1,4-Phenylen, Pyrimidin-2,5-diyl oder Pyridin-2,5-diyl bedeutet, auch Fluor, Chlor oder Cyano darstellt.

2. Verbindungen nach Anspruch 1 dadurch gekennzeichnet, dass R² Cyano bedeutet.

3. Verbindungen nach Anspruch 1, dadurch gekennzeichnet, dass R² Alkyl, Alkoxy, Alkenyl oder Alkenyloxy mit 1 bzw. 2 bis 7 Kohlenstoffatomen bedeutet.

4. Verbindungen gemäss einem der Ansprüche 1 bis 3, dadurch gekennzeichnet, dass n entweder 0 oder 1 ist.

5. Verbindungen gemäss einem der Ansprüche 1, 2 oder 3 der allgemeinen Formeln 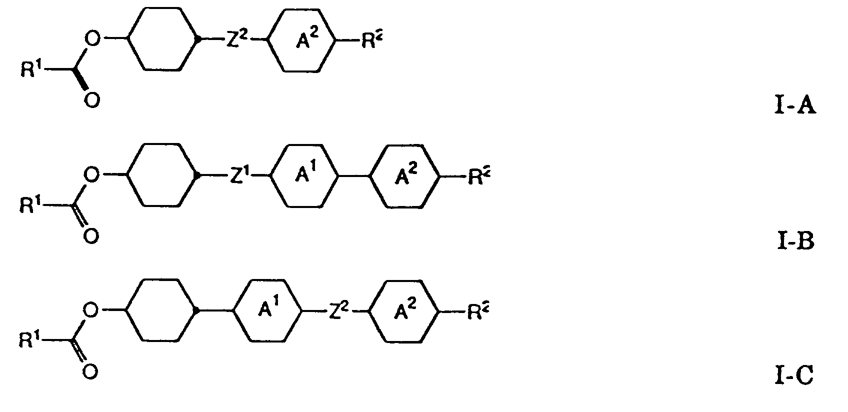 worin
R¹ 1E-Alkenyl mit 3 bis 7 Kohlenstoffatomen bedeutet,
A¹ unsubstituiertes oder mit Fluor substituiertes 1,4-Phenylen, oder trans-1,4-Cyclohexylen bedeutet,
A² unsubstituiertes oder mit Fluor substituiertes 1,4-Phenylen, Pyrimidin-2,5-diyl, Pyridin-2,5-diyl oder trans-1,4-Cyclohexylen bedeutet,
Z¹, Z² unabhängig voneinander eine einfache Kovalenzbindung, -CH₂CH₂-, -CH₂O-, -OCH₂-, -COO- oder -OOC- darstellen, und
R² Alkyl, Alkoxy, Alkenyl oder Alkenyloxy mit 1 bzw. 2 bis 7 Kohlenstoffatomen oder,
in dem Fall daß A¹ oder A² ein unsubstituierter oder mit Fluor substituiertes 1,4-Phenylen, Pyrimidin-2,5-diyl oder Pyridin-2,5-diyl bedeutet, auch Fluor, Chlor oder Cyano darstellt.

6. Verbindungen gemäss einem der Ansprüche 1 bis 5, dadurch gekennzeichnet, dass eine der Gruppen Z¹ und Z² eine einfache Kovalenzbindung und die andere der Gruppen Z¹ und Z² eine einfache Kovalenzbindung, -CH₂CH₂-, -COO- oder -OOC- bedeuten.

7. Verbindungen gemäss einem der Ansprüche 2, 4 bis 6 der Formeln 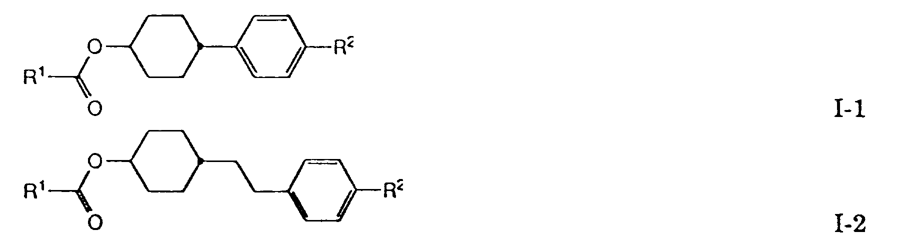 worin R¹ 1E-Propenyl oder 1E-Butenyl bedeutet; und R² Cyano bedeutet.

8. Verbindungen gemäss einem der Ansprüche 4 bis 6 der Formeln 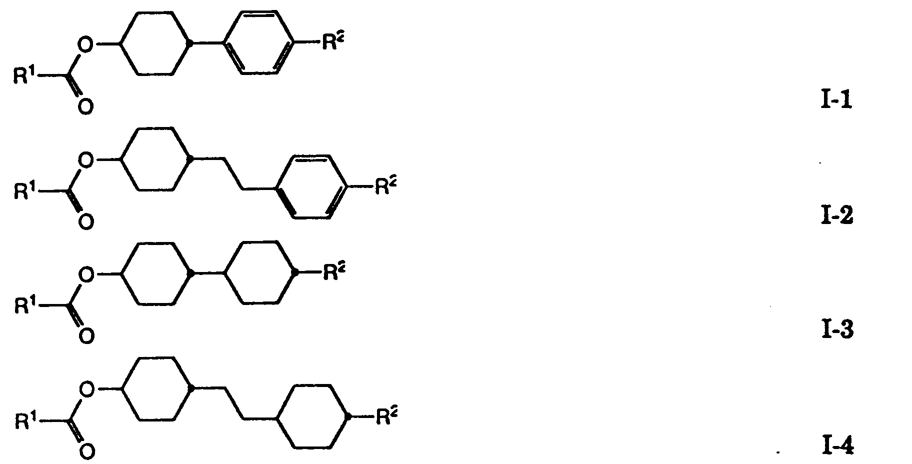 worin R¹ 1E-Propenyl oder 1E-Butenyl bedeutet; und R² Alkyl oder Alkoxy mit 1 bis 7 Kohlenstoffatomen bedeutet.

9. Verbindungen gemäss einem der Ansprüche 2, 4 bis 7 der Formeln 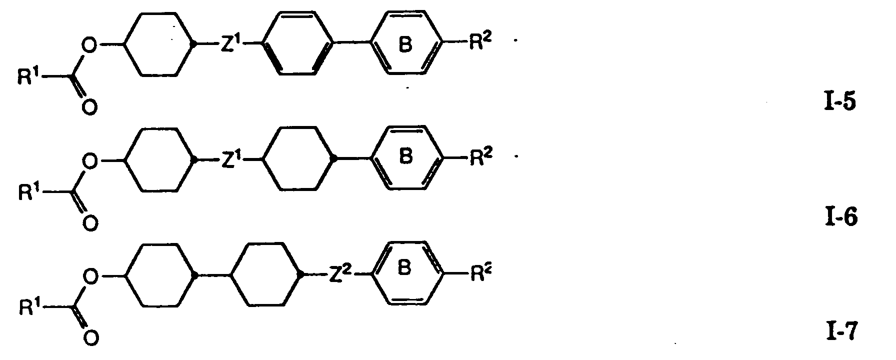 worin
R¹ 1E-Propenyl oder 1E-Butenyl darstellt,
Z¹, Z² eine einfache Kovalenzbindung, -CH₂CH₂-, -COO- oder -OOC-bedeuten;
Ring B 1,4-Phenylen, 2-Fluor-1,4-phenylen (dh. in ortho-Stellung zum Rest R²), 2,3-Difluor-1,4-phenylen, 2,6-Difluor-1,4-phenylen darstellt, und
R² Cyano bedeutet.

10. Verbindungen gemäss Anspruch 9, dadurch gekennzeichnet, dass Z¹ bzw. Z² eine einfache Kovalenzbindung oder -CH₂CH₂- bedeuten.

11. Flüssigkristallines Gemisch mit mindestens 2 Komponenten, dadurch gekennzeichnet, dass mindestens eine Komponente eine Verbindung der in Anspruch 1 definierten Formel I ist.

12. Verwendung der Verbindungen der in Anspruch 1 definierten Formel I für elektro-optische Zwecke.

## Claims

1. Compounds of the general formula 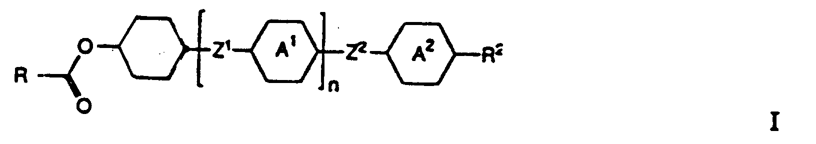 in which
R¹ is a 1E-alkenyl group having 3 to 7 carbon atoms;
A¹ and A², independently of one another, are unsubstituted or halogen-substituted 1,4-phenylene, in which, if unsubstituted, 1 or 2 CH groups may, if desired, be replaced by nitrogen, or are trans-1,4-cyclohexylene or 1,3-dioxane-2,5-diyl;
Z¹ and Z² independently of one another, are a single covalent bond, -CH₂CH₂-, -COO-, -OOC-, -OCH₂-, -CH₂O-, -C≡C-, -(CH₂)₄-, -O(CH₂)₃-, -(CH₂)₃O- or the trans-form of -OCH₂CH=CH-, -CH=CHCH₂O-, 0(CH₂)₂CH=CH- or -CH=CH(CH₂)₂-;
n is either 0, 1 or 2; and
R² is alkyl, alkoxy, alkenyl or alkenyloxy having 1 or 2 to 7 carbon atoms respectively, or, in the case where A¹ or A² is unsubstituted or fluorine-substituted 1,4-phenylene, pyrimidine-2,5-diyl or pyridine-2,5-diyl, R² is alternatively fluorine, chlorine or cyano.

2. Compounds according to Claim 1, characterized in that R² is cyano.

3. Compounds according to Claim 1, characterized in that R² is alkyl, alkoxy, alkenyl or alkenyloxy having 1 or 2 to 7 carbon atoms respectively.

4. Compounds according to one of Claims 1 to 3, characterized in that n is either 0 or 1.

5. Compounds according to one of Claims 1, 2 or 3, of the general formulae 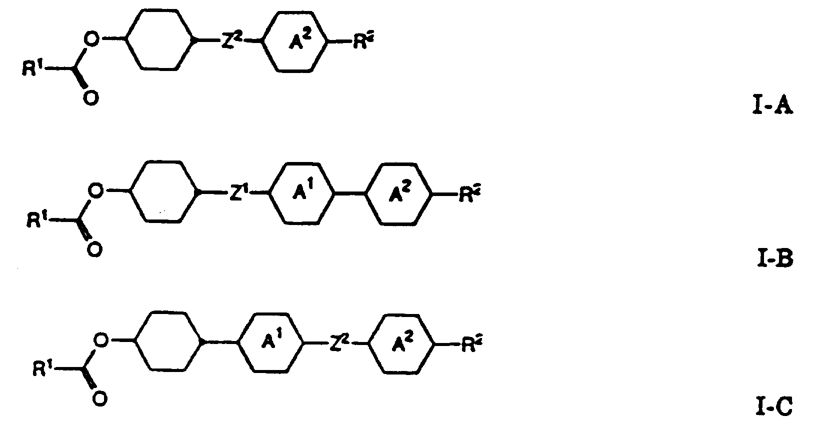 in which
R¹ is 1E-alkenyl having 3 to 7 carbon atoms,
A¹ is unsubstituted or fluorine-substituted 1,4-phenylene, or is trans-1,4-cyclohexylene,
A² is unsubstituted or fluorine-substituted 1,4-phenylene, pyrimidine-2,5-diyl, pyridine-2,5-diyl or trans-1,4-cyclohexylene,
Z¹ and Z² independently of one another, are a single covalent bond, -CH₂CH₂-, -CH₂O-, -OCH₂-, -COO- or -OOC-, and
R² is alkyl, alkoxy, alkenyl or alkenyloxy having 1 or 2 to 7 carbon atoms respectively, or, in the case where A¹ or A² is unsubstituted or fluorine-substituted 1,4-phenylene, pyrimidine-2,5-diyl or pyridine-2,5-diyl, R² is alternatively fluorine, chlorine or cyano.

6. Compounds according to one of Claims 1 to 5, characterized in that one of the groups Z¹ and Z² is a single covalent bond and the other of the groups Z¹ and Z² is a single covalent bond, -CH₂CH₂-, -COO- or -OOC-.

7. Compounds according to one of Claims 2 and 4 to 6, of the formulae 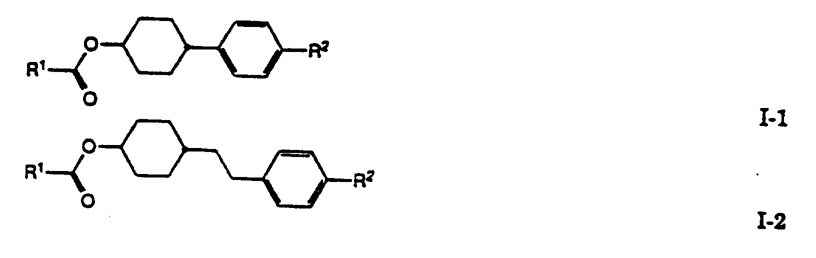 in which R¹ is 1E-propenyl or 1E-butenyl; and R² is cyano.

8. Compounds according to one of Claims 4 to 6, of the formulae 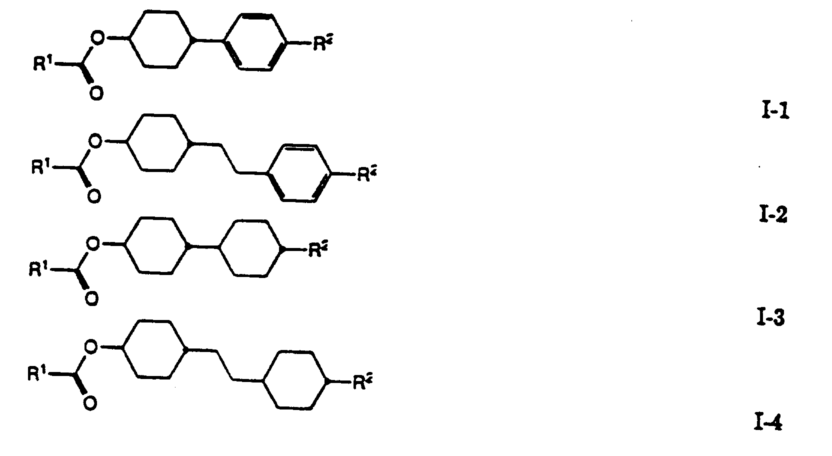 in which R¹ is 1E-propenyl or 1E-butenyl; and R² is alkyl or alkoxyl having 1 to 7 carbon atoms.

9. Compounds according to one of Claims 2 and 4 to 7, of the formulae 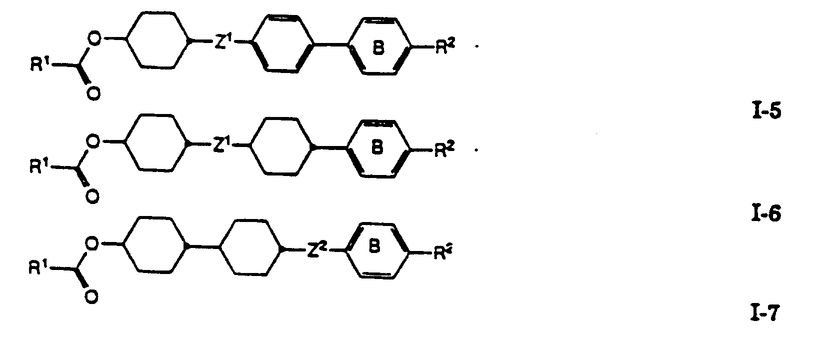 in which
R¹ is 1E-propenyl or 1E-butenyl,
Z¹ and Z² are a single covalent bond, -CH₂CH₂-, -COO- or -OOC-;
Ring B is 1,4-phenylene, 2-fluoro-1,4-phenylene (i.e. in the ortho-position to the radical R²), 2,3-difluoro-1,4-phenylene, or 2,6-difluoro-1,4-phenylene, and
R² is cyano.

10. Compounds according to Claim 9, characterized in that Z¹ or Z² is a single covalent bond or -CH₂CH₂-.

11. Liquid-crystalline mixture having at least 2 components, characterized in that at least one component is a compound of the formula I defined in Claim 1.

12. Use of the compounds of the formula I defined in Claim 1 for electro-optical purposes.

## Revendications

1. Composés de formule générale 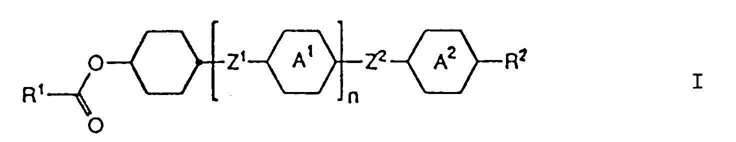 dans laquelle
R¹ représente un groupe 1E-alcényle en C₃-C₇ ;
A¹ et A² représentent chacun, indépendamment l'un de l'autre, un groupe 1,4-phénylène non substitué ou substitué par des halogènes, et dans lequel, lorsqu'il est non substitué, un ou deux groupes CH peuvent le cas échéant être remplacés par l'azote, ou un groupe trans-1,4-cyclohexylène ou trans-1,3-dioxanne-2,5-diyle ;
Z¹ et Z² représentent chacun, indépendamment l'un de l'autre, une liaison covalente simple, un groupe -CH₂CH₂-, -COO-, -OOC-, -OCH₂-, -CH₂O-, -C≡C-, -(CH₂)₄-, -O(CH₂)₃-, -(CH₂)₃O- ou la forme trans de l'un des groupes -OCH₂CH=CH-, -CH=CHCH₂O-, -(CH₂)₂CH=CH- ou -CH=CH(CH₂)₂- ;
n est égal à 0, 1 ou 2 ; et
R² représente un groupe alkyle, alcoxy, alcényle ou alcényloxy contenant un ou deux à sept atomes de carbone, ou bien
lorsque A¹ ou A² représente un groupe 1,4-phénylène, pyrimidine-2,5-diyle ou pyridine-2,5-diyle non substitué ou substitué par le fluor, R² peut également représenter le fluor, le chlore ou un groupe cyano.

2. Composés selon revendication 1, caractérisés en ce que R² représente un groupe cyano.

3. Composés selon revendication 1, caractérisés en ce que R² représente un groupe alkyle, alcoxy, alcényle ou alcényloxy contenant un ou deux à sept atomes de carbone.

4. Composés selon l'une des revendications 1 à 3, caractérisés en ce que n est égal à 0 ou 1.

5. Composés selon l'une des revendications 1, 2 ou 3, répondant aux formules générales 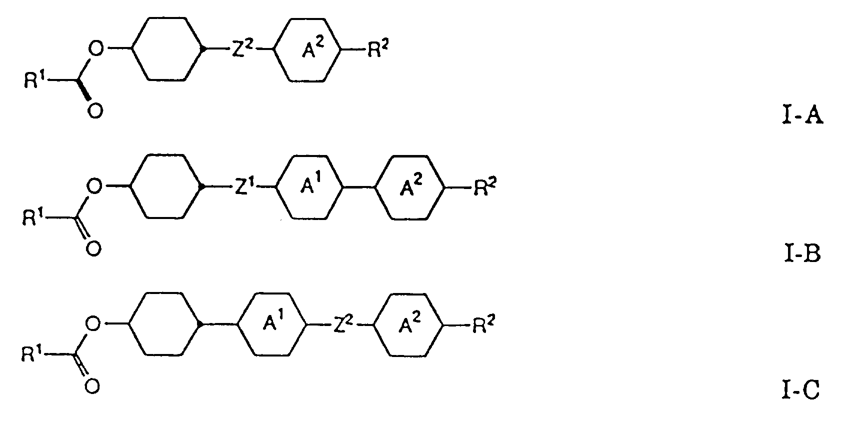 dans lesquelles
R¹ représente un groupe 1E-alcényle en C₃-C₇,
A¹ représente un groupe 1,4-phénylène non substitué par le fluor, ou un groupe trans-1,4-cyclohexylène,
A² représente un groupe 1,4-phénylène, pyrimidine-2,5-diyle, pyridine-2,5-diyle ou trans-1,4-cyclohexylène non substitué ou substitué par le fluor,
Z¹ et Z² représentent chacun, indépendamment l'un de l'autre, une liaison covalente, un groupe -CH₂CH₂-, -CH₂O-, -OCH₂-, -COO- ou -OOC- et
R² représente un groupe alkyle, alcoxy, alcényle ou alcényloxy contenant un ou deux à sept atomes de carbone, ou bien
lorsque A¹ ou A² représente un groupe 1,4-phénylène, pyrimidine-2,5-diyle ou pyridine-2,5-diyle non substitué ou substitué par le fluor, R² peut également représenter le fluor, le chlore ou un groupe cyano.

6. Composés selon l'une des revendications 1 à 5, caractérisés en ce que l'un des symboles Z¹ et Z² représente une liaison covalente simple et l'autre une liaison covalente simple, un groupe -CH₂CH₂-, -COO- ou -OOC-.

7. Composés selon l'une des revendications 2 et 4 à 6, de formules 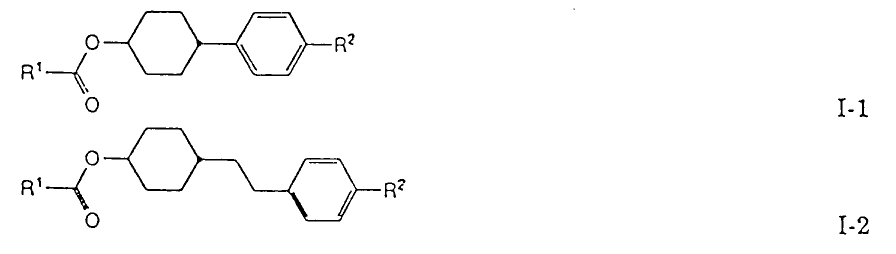 dans lesquelles R¹ représente un groupe 1E-propényle ou 1E-butényle, et R² un groupe cyano.

8. Composés selon l'une des revendications 4 à 6, de formules 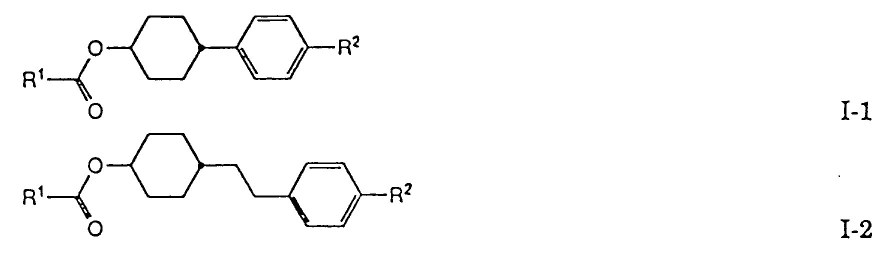 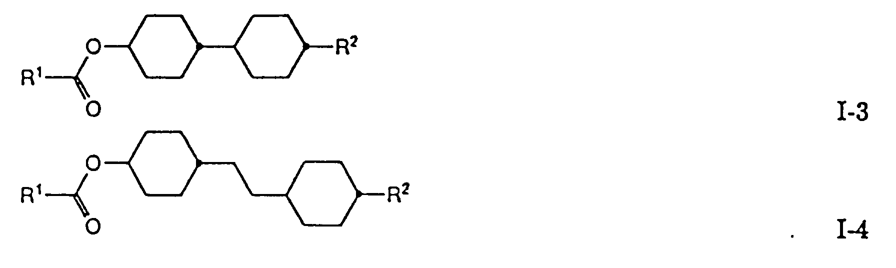 dans lesquelles R¹ représente un groupe 1E-propényle ou 1E-butényle et R² représente un groupe alkyle ou alcoxy en C₁-C₇.

9. Composés selon l'une des revendications 2 et 4 à 7, de formules 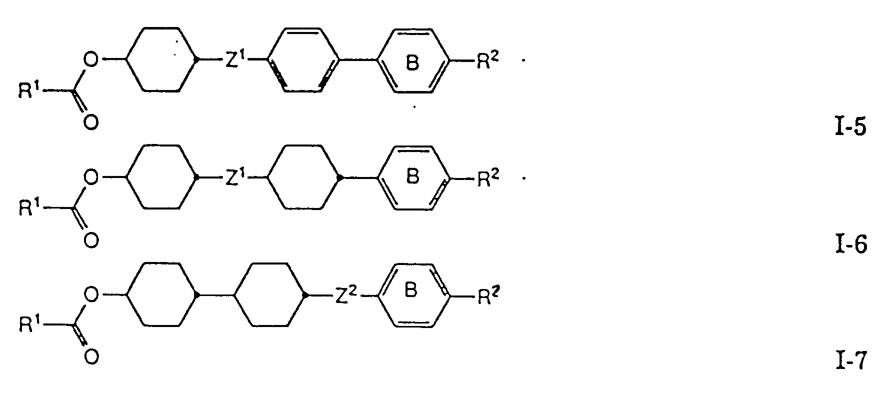 dans lesquelles
R¹ représente un groupe 1E-propényle ou 1E-butényle,
Z¹ et Z² représentent chacun une liaison covalente simple, un groupe -CH₂CH₂-, -COO- ou -OOC- ;
le cycle B est un cycle 1,4-phénylène, 2-fluoro-1,4-phénylène (c'est-à-dire en position ortho par rapport au groupe R²), 2,3-difluoro-1,4-phénylène, 2,6-difluoro-1,4-phénylène
et
R² représente un groupe cyano.

10. Composés selon revendication 9, caractérisés en ce que l'un des symboles Z¹ et Z² représente une liaison covalente simple ou un groupe -CH₂CH₂-.

11. Mélange à cristaux liquides à au moins deux composants, caractérisé en ce que l'un au moins des composants est un composé de formule I de la revendication 1.

12. Utilisation des composés de formule I de la revendication 1 dans des applications électro-optiques.
